# EUROPÄISCHE PATENTSCHRIFT

(11) **EP 0 345 409 B1**
(45) Veröffentlichungstag und Bekanntmachung des Hinweises auf die Patenterteilung: **05.06.2002**
(21) Anmeldenummer: 89100395.6
(22) Anmeldetag: 11.01.1989
(51) Int. Cl.: D06F 39/02, D06F 58/20

(54) **Dosiervorrichtung zur Aufnahme und Abgabe eines Wäschebehandlungsmittels**
Dosing device for holding and dispensing a treatment product for the laundry
Dispositif doseur pour retenir et distribuer un produit de traitement pour le linge

(30) Priorität: 15.02.1988 DE 8801919 U; 12.10.1988 DE 8812795 U
(43) Veröffentlichungstag der Anmeldung: 13.12.1989
(60) Teilanmeldung: 00120798.4
(73) Patentinhaber: Henkel Kommanditgesellschaft auf Aktien, 40191 Düsseldorf (DE)
(72) Erfinder: Weber, Rudolf, D-4000 Düsseldorf 13 (DE); Jobs, Jörn, D-4060 Viersen 12 (DE)
(74) Vertreter: Rieder, Hans-Joachim, Dr.

(56) Entgegenhaltungen:
- FR-A- 340 720
- US-A- 1 922 085
- US-A- 3 947 971
- US-A- 4 532 722

## Beschreibung

Die Erfindung betrifft eine Dosiervorrichtung zur Aufnahme und Abgabe eines Wäschebehandlungsmittels in einer Waschmaschine, in einem Wäschetrockner oder dergleichen, mit einem das Wäschebehandlungsmittel aufnehmenden Speicher.

Bei der Verwendung von Flüssig-Waschmittel ist man dazu übergegangen, dieses dosiert in ein Speichergefäß einzufüllen, das mit der zu behandelnden Wäsche in die Trommel einer Waschmaschine eingebracht wird. Durch die Rotation der Trommel tritt während des Waschvorganges das Flüssig-Waschmittel aus einer Austrittsöffnung des Speicherbehälters aus. Der Speicherbehälter besteht aus starrem Kunststoffmaterial. Bei nicht sachgemäßer Anwendung kann es vorkommen, dass eine Entleerung des Speicherbehälters gleich zu Beginn des Waschvorganges - wenn noch kein oder nur wenig Wasser in der Waschmaschine vorhanden ist - oberhalb des Laugenstutzens der Waschmaschine erfolgt, so dass das Flüssig-Waschmittel in den Laugenstutzen eintritt und für den Waschvorgang nicht mehr oder nur teilweise zur Verfügung steht.

Die US-A 3,947,971 beschreibt eine Dosiervorrichtung mit einem Paar flexibler, poröser, offenenzelliger Schaumstoffplatten, welche aufeinander liegend derart miteinander befestigt sind, dass ein zu einem Rand hin unverschlossener Spalt ausgebildet ist, welcher zum Einführen einer Wäschebehandlungsmittel enthaltenden Tablette geöffnet werden kann.

Aus der US-A 4,532,722 ist eine Vorrichtung zur Abgabe eines Konditionierungsmittels für Textilien in einem Wäschetrockner bekannt. Eine solche Vorrichtung ist mit einem absorbierendem Material gefüllt, das weiter mit einem flüssigen Textil-Konditionierungsmittel bis zur Sättigung übergossen ist. Das Konditionierungsmittel soll nur dadurch abgegeben werden, dass Heißluft, die den Wäschetrockner durchsetzt, zur Verdampfung des Konditionierungsmittels führt. Der Dampf erst tritt dann durch Öffnungen in dem Behältnis aus, um die gewünschten Duft-Eigenschaften auf das Textil zu übertragen. Zu diesem Zweck ist die Wandung des Behältnisses aus einem lediglich für Wasserdampf, nicht aber für Wasser durchlässigen Textil gebildet.

Der Erfindung liegt die Aufgabe zugrunde, eine Dosiervorrichtung der eingangs genannten Art zu schaffen, bei der, unter Vermeidung der genannten Nachteile, Verluste der Waschmittelsubstanz vermieden werden. Gleichzeitig soll die Möglichkeit gegeben sein, mit den gleichen Dosiergefäßen pulver- und pastenförmige Produkte verlustfrei zu dosieren.

Diese Aufgabe ist durch den Gegenstand des Anspruches 1 gelöst. Aufgrund der Rückhaltefähigkeit einer derartigen Hülle kann auch bei einer unsachgemäßen Vorgehensweise das im Speicher befindliche Waschmittel oder dergleichen nicht direkt in umfangreicher Menge austreten. Diese Rückhaltfähigkeit resultiert aus einer gewissen Rückhaltewirkung der Zellstruktur gegenüber dem Wäschebehandlungsmittel. Das Wasser hingegen kann jedoch die Zellstruktur leicht durchdringen und dann das verdünnte bzw. gelöste Wäschebehandlungsmittel aus der Hülle herausspülen und/oder die Anordnung ist so getroffen, dass die Wäschebehandlungspaste aufgrund ihrer relativen Dickflüssigkeit nur langsam die Zellstruktur durchdringt (hindurchdiffundiert), so dass über einen langen Zeitraum kontinuierlich das Behandlungsmittel an das Waschwasser abgegeben wird, was äußerst förderlich für das Waschergebnis ist. Es ist damit stets sichergestellt, dass erst mit Zulauf des Waschwassers ein nennenswertes Austreten des Wäschebehandlungsmittels aus der erfindungsgemäßen Vorrichtung erfolgt. Insbesondere ist die Zellstruktur flexibel ausgebildet, indem sie vorzugsweise aus einem textilen Flächengebilde besteht, wodurch sich eine hohe Wäschefreundlichkeit und ein "geräuschloser" Waschvorgang ergibt. Die letzteren Eigenschaften sind bei den bekannten aus festem Kunststoff bestehenden Dosiervorrichtungen nicht gegeben, da beim Waschvorgang eine erhebliche Reibung an dem harten Speicherbehälter auftritt und der Behälter beim Anstoßen an die Trommelwandungen ein störendes Geräusch auslöst.

Nach einer Weiterbildung der Erfindung ist vorgesehen, dass die Hülle als Beutel ausgebildet ist.

Alternativ kann jedoch auch vorgesehen sein, dass die Hülle als kurvertartige Tasche ausgebildet ist.

Möglich ist es bevorzugt ferner für die Hülle Schlingenware, z. B. Frottier oder Frottee, Strickware oder Damast zu verwenden.

Für den Verschluss der Hülle besteht die Möglichkeit ein Klettband, einem Kordelzug, einen Reißverschluss, ein elastisches Band oder einen Kleberverschluss einzusetzen. Bevorzugt kommt das Klettband zum Einsatz. Sofern der Kleberverschluss gewählt ist, ist es von Vorteil, wenn dieser temperaturabhängig reagiert, d. h., dass ein Öffnen des Verschlusses erfolgt, sobald das Waschwasser eine bestimmte Temperatur erreicht hat.

Die Anordnung kann so getroffen sein, dass ein Speicher als separate, in die Hülle einsetzbare, flüssigkeitsundurchlässige Messkappe ausgebildet ist. Als Messkappe kann bspw. die Verschlusskappe einer Flüssig-Waschmittelvorratsflasche dienen.

Nach einer anderen Ausführungsform der Erfindung ist es jedoch auch möglich, dass der Speicher von einer behälterartig ausgebildeten, flüssigkeitsundurchlässigen Folie gebildet ist. Vorzugsweise ist die Folie im Inneren der Hülle angeordnet. Wird der oben beschriebene, temperaturabhängige Kleberverschluss eingesetzt, so kann die Hülle insgesamt mit der Folie versehen sein. In diesem Falle würde erst nach Öffnung des Verschlusses ein Austreten des Wäschebehandlungsmittels - das flüssig oder auch pulverförmig sein kann - durch entsprechende Walkarbeit durch die Trommeldrehung der Maschine erfolgen.

Die Folie kann neben der Hülle eine separate Wandung bilden, so dass eine Zweistückigkeit vorliegt. Alternativ ist es nach einer Weiterbildung der Erfindung jedoch auch möglich, dass die Folie von einer Beschichtung der Haut der Hülle gebildet ist. Mithin liegt dann hier eine Einstückigkeit vor.

Besonders vorteilhaft ist es, wenn die Folie im Bodenbereich der Hülle angeordnet ist und der Verschlussbe reich der Hülle keine Folie aufweist. Mithin ist angrenzend an den Verschluss der Hülle ein folienfreier Bereich ausgebildet. Diese Ausgestaltung gestattet das Einfüllen des flüssigen Wäschebehandlungsmittels in dosierter Form in den Bodenbereich bzw. unteren Bereich der durch die Folie dann dort flüssigkeitsundurchlässigen Hülle. Nach dem Verschluss der Hülle wird diesedann zusammen mit der zu behandelnden Wäsche in die Trommel der Waschmaschine gegeben. Ein sofortiges Auslaufen der Wäschebehandlungsflüssigkeit ist vermieden, da der im Bereich des Verschlusses gelegene Abschnitt der Hülle, aufgrund seiner offenporigen Zellstruktur die oben beschriebene Rückhaltefähigkeit besitzt. Erst wenn während des Waschprozesses Wasser hinzutritt und eine Walkarbeit erfolgt, wird das Flüssig-Waschmittel oder dergleichen aus der erfindungsgemäßen Vorrichtung "ausgewaschen" und gelangt somit aktiv in den Waschprozess.

Bei offenporigem Schaumstoff erhält man entsprechend der Offenporigkeit über einen wählbaren Zeitraum eine kontinuierliche Abgabe bzw. ein kontinuierliches Austreten des Wäschebehandlungsmittels. Der offenporige Schaumstoff zur Bildung der Hülle gestattet es, entsprechende Formen zu erzeugen, um ein besonders gutes Unterbringen des das Wäschebehandlungsmittel aufnehmenden Speichers zu erhalten.

Auch sind - wie oben erwähnt - entsprechende Lösungen mit Schaumstoff und Folie oder be- bzw. verhautetem Schaumstoff möglich.

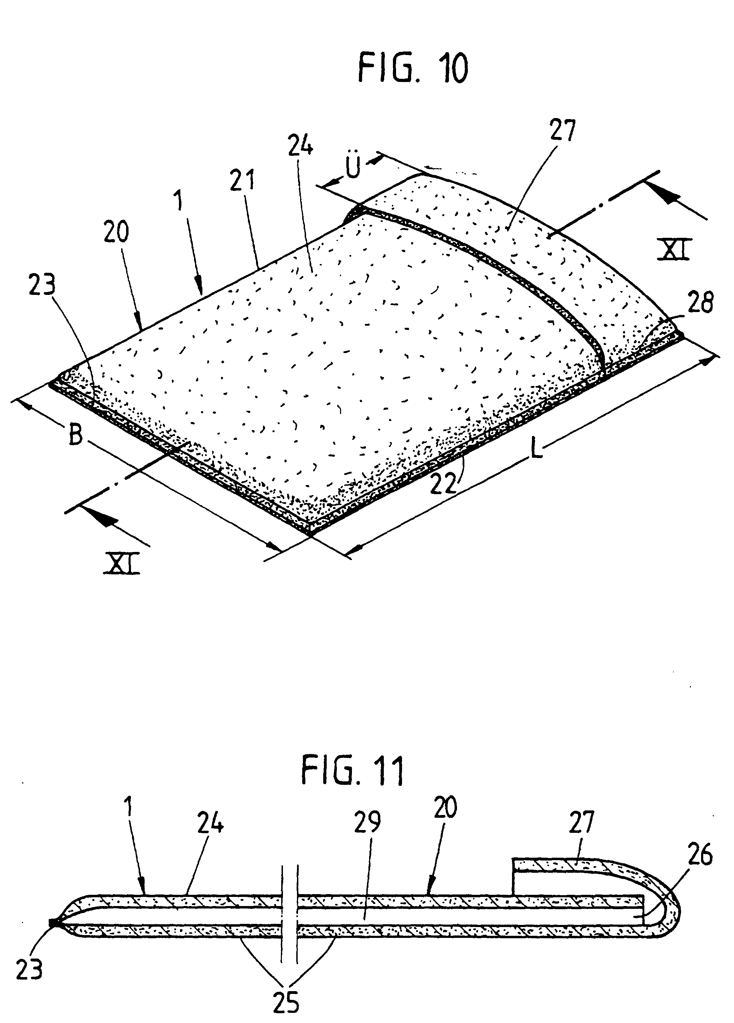

Es ist jedoch auch denkbar, daß der Speicher von einem verschließbaren Behältnis aus offenporigem Schaumstoff gebildet ist. Dadurch erhöht sich sogar sein Einsatzbereich. Er kann sowohl dazu dienen, einen Wäschebehandlungsflüssigkeit enthaltenden Behälter aufzunehmen als auch Waschpulver. Im letzten Fall kann der Behälter entfallen. Das entsprechende Schaumstoff-Behältnis kann in den verschiedensten Ausführungsformen erstellt werden, wie beispielsweise in Beutelform, Kuvertform (z.B. mit auf links gearbeitetem Hotelverschluß, wie dieser in Fig. 11 gezeigt ist). Das Einfüllen von Waschpulver in das Schaumstoff-Behältnis und Einlegen zwischen die Wäsche erfolgt in der vorbeschriebenen Weise. Nach dem Einlegen und Einschalten des Waschvorganges dringt das Waschwasser in den Innenraum des Behältnisses ein, vermischt sich mit dem Waschpulver, welche Lösung vorzugsweise nach einigen Minuten das Behältnis verläßt. Bedingt durch den Einsatz von Schaumstoff zur Schaffung des Behältnisses lassen sich die Herstellungskosten sehr niedrig halten.

Um den verschiedenen Anforderungen bei einem Waschprozeß gerecht zu werden, ist der Schaumstoff bis mindestens 95° C temperaturbeständig. Das Schaumstoff-Behältnis ist daher für jeden angestrebten Waschprozeß geeignet.

Als dauerhaft und kochbeständig hat sich insbesondere offenporiger Polyurethan-Schaumstoff erwiesen. Die Dicke des Schaumstoffes zur Schaffung des Behältnisses liegt dabei zwischen 1 bis 6mm. In Versuchen hat sich als günstig erwiesen eine Dicke von 3-4mm zu wählen. Vorzugsweise löst sich das Waschpulver in einem geschlossenen Beutel aus offenporigem Polyurethan in ca. 2 bis 3min. auf und gelangt dabei in das Waschwasser.

Diesbezüglich zeichnet sich eine vorteilhafte Bauform dadurch aus, daß die Hülle bzw. das Behältnis von einer Schaumstofftasche gebildet ist. Die Taschenform kommt einer einfachen, kostensparenden Herstellung entgegen. Auch eignet sie sich dazu, entweder Waschpulver oder einen Wäschebehandlungsflüssigkeit enthaltenden Behälter zu umhüllen.

Insbesondere bietet sich eine solche Bauform an, die dadurch gekennzeichnet ist, daß die Schaumstofftasche zwei rechteckförmige, deckend zueinander angeordnete, an drei Randseiten miteinander verbundene Taschenwände aufweist. Hierdurch ist ein weitgehend verlustfreier Zuschnitt zur Bildung der Schaumstofftasche möglich. Darüber hinaus ist die Verbindung an geradlinigen Randseiten vornehmbar.

Um ein ungewolltes Austreten des Tascheninhaltes vor der Durchflutung zu verhindern, geht von der einen Taschenwand eine umgelegte, die Taschenöffnung überfangende Verschlußlasche aus, deren Schmalkanten mit den entsprechenden Bereichen der Randseiten verbunden sind. Dies kann gleichzeitig bei der Randseitenverbindung geschehen. Insbesondere kommt einer günstigen Fertigung die Tatsache entgegen, daß die Randseiten zusammen mit der jeweils zugehörigen Schmalkante miteinander verschweißt sind. Zusätzliches Material zur Randseiten- und Schmalkantenverbindung ist daher nicht erforderlich, da die Schmelzfähigkeit des Schaumstoffes ausgenutzt wird. Für die Randseitenverschweißung bietet sich insbesondere eine Hochfrequenzschweißung an.

Wenn die Verschlußlasche einstückig mit der zugehörigen Taschenwand ausgebildet ist, kann zur Bildung der Schaumstofftasche von einem einzigen Zuschnitt ausgegangen werden.

Gebrauchsvorteile ergeben sich dadurch, daß die Schaumstofftasche ein Format von etwa 20 cm Länge und 15 cm Breite aufweist. Daher kann sie genügend Waschmittel für einen Waschvorgang in einer Waschtrommel aufnehmen. Insbesondere bietet sich diese Form und Größe für eine Waschpulverdosierung an und könnte als sogenanntes "Waschmittelkissen" zum Einsatz kommen. Das Befüllen geschieht dabei über die an der einen Breitseite der Schaumstofftasche ausgebildete Taschenöffnung.

Das Austreten des in der Schaumstofftasche befindlichen Waschpulvers durch die Taschenöffnung wird dadurch verhindert, daß die Verschlußlasche einen Überstand über die Taschenöffnung von etwa 3-6cm aufweist.

Die Zeichnungen veranschaulichen die Erfindung anhand mehrerer Ausführungsbeispiele und zwar zeigt:
- Fig. 1: eine aus offenporiger Zellstruktur bestehende Hülle zur Aufnahme einer Meßkappe,
- Fig. 2: die Hülle gem. Fig. 1 mit in ihrem Innern aufgenommer Meßkappe,
- Fig. 3: einen Längsschnitt entlang der Linie III - III in Fig. 2,
- Fig. 4: eine Draufsicht auf eine als kuvertartige Tasche ausgebildete Hülle,
- Fig. 5: eine Schnittansicht entlang der Linie V - V in Fig. 4,
- Fig. 6: eine Ansicht einer als Beutel ausgebildeten Hülle, deren Verschluß als Kordelzug ausgebildet ist,
- Fig. 7: eine Draufsicht auf ein weiteres Ausführungsbeispiel eines Beutels, der einen quadratischen Boden aufweist,
- Fig. 8: ein weiteres Ausführungsbeispiel einer Hülle, die im unteren Bereich mit einer flüssigkeitsundurchlässigen Folie versehen ist,
- Fig. 9: einen Querschnitt durch die Hülle gemäß Fig. 8 entlang der Linie IX-IX,
- Fig. 10: in perspektivischer Darstellung ein weiteres Ausführungsbeispiel einer in Form einer Schaumstofftasche ausgebildeten Hülle und
- Fig. 11: den Schnitt nach der Linie XI-XI in Fig. 10.

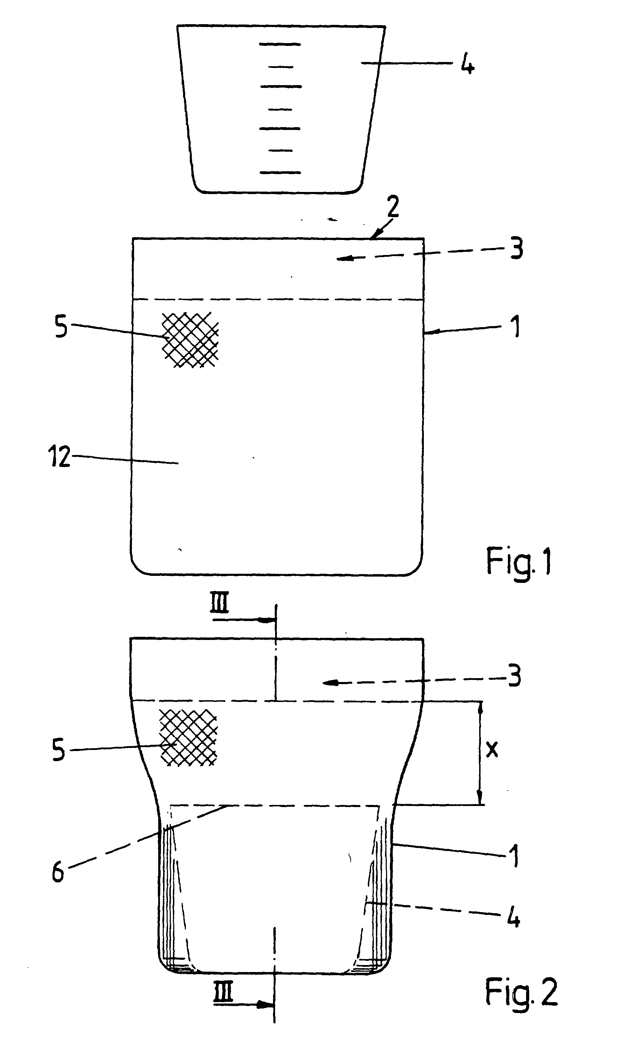

Die erfindungsgemäße Dosiervorrichtung besteht gemäß Fig. 1 aus einer Hülle 1, die eine Öffnung 2 aufweist, welche mittels eines Klettverschlusses 3 verschließbar ist. Die Abmessungen der Hülle 1 sind derart gewählt, daß durch die Öffnung 2 eine Meßkappe 4 in das Innere der Hülle eingebracht werden kann (vergleiche Fig. 2). Die Hülle besteht aus einer flexiblen, offenporigen Zellstruktur 5, die in Abhängigkeit von ihrem Material und ihrer Dicke die oben beschriebene Rückhaltfähigkeit besitzt. Als Zellstrukur kann Gewebe, Gewirke, Vlies, Schlingenware, z. B. Frottier oder Frottee, Strickware, Damast und vorzugsweise auch Schaumstoff zum Einsatz kommen.

Gemäß Fig. 2 ist sichergestellt, daß oberhalb des oberen Becherrandes 6 ein Abstand x bis zum Klettverschluß 3 besteht, mit anderen Worten die Beutellänge - ohne Verschluß - ist größer als die Höhe der Meßkappe 4. Hierdurch ist ein Austrittsfreiraum geschaffen.

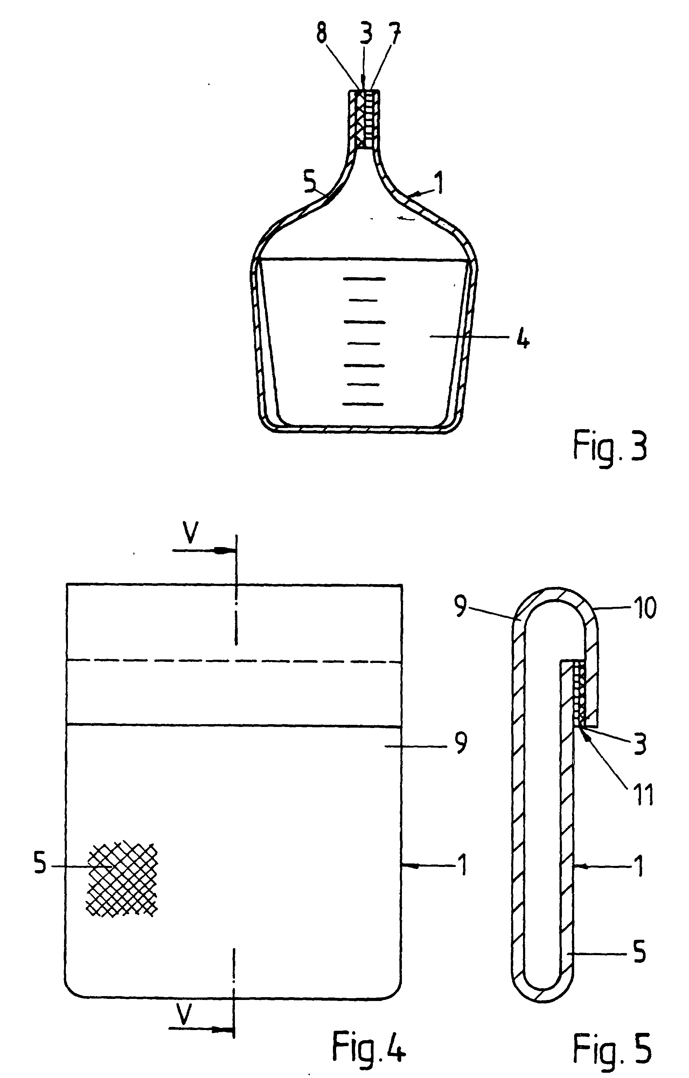

Die Fig. 3 verdeutlicht nochmal den Aufbau der erfindungsgemäßen Vorrichtung. Deutlich ist der aus zwei Bändern 7,8 bestehende Kettverschluß 3 erkennbar.

Bei der Anwendung wird wie folgt vorgegangen:

Zunächst wird eine entsprechende Menge eines flüssigen Wäschebehandlungsmittels in die Meßkappe 4 gegeben. Anschließend wird die Meßkappe 4 durch die Öffnung 2 in das Innere der Hülle 1 eingebracht und schließlich die Öffnung 2 durch den Klettverschluß 3 verschlossen. Zusammen mit den zu behandelnden Wäschestücken wird dann die erfindungsgemäße Vorrichtung in die Trommel einer Waschmaschine oder dergleichen eingebracht. Durch die Trommelbewegung läuft die Wäschebehandlungsflüssigkeit aus der Meßkappe 4 heraus, was insbesondere durch den mittels des Abstandes x gebildeten Freiraum möglich ist. Die offenporige Zellstruktur 5 der Hülle 1 saugt die Behandlungsflüssigkeit im wesentlichen, zumindest jedoch teilweise, auf. Wenn nunmehr die Waschmaschine mit Wasser gefüllt wird, erfolgt durch die Walkbewegung ein Austreten bzw.Auswaschen des Flüssig-Waschmittels und wird damit für den Waschprozeß aktiviert. Nach Beendigung des Waschvorganges kann die erfindungsgemäße Hülle 1 wieder verwendet werden. Durch geeignete Materialwahl von Meßkappe 4 und Hülle 1 ist es möglich, die Vorrichtung auch selbst bei 95° Waschtemperatur einzusetzen. Wesentlich ist ferner, daß die zumeist etwas scharfkantige Meßkappe keinen direkten Textilkontakt mit den zu behandelnden Wäschestücken hat, wodurch sich eine äußerst wäschefreundliche Behandlungsweise ergibt.

Die Fig. 4 zeigt ein weiteres Ausführungsbeispiel der erfindungsgemäßen Hülle 1, die gegenüber dem zuvor beschriebenen Ausführungsbeispiel nicht beutelförmig, sondern als kuvertartige Tasche 9 ausgebildet ist. Hierzu weist die Tasche 9 eine Überschlagslasche 10 auf, die mit einem Klettverschluß 3 zum Schließen der Taschenöffnung 11 festlegbar ist. Auch in diese Tasche 9 wird - wie zuvor beschrieben - ein entsprechender Meßbecher 4 eingebracht; der Einsatz erfolgt in gleicher Weise, wie zuvor beschrieben.

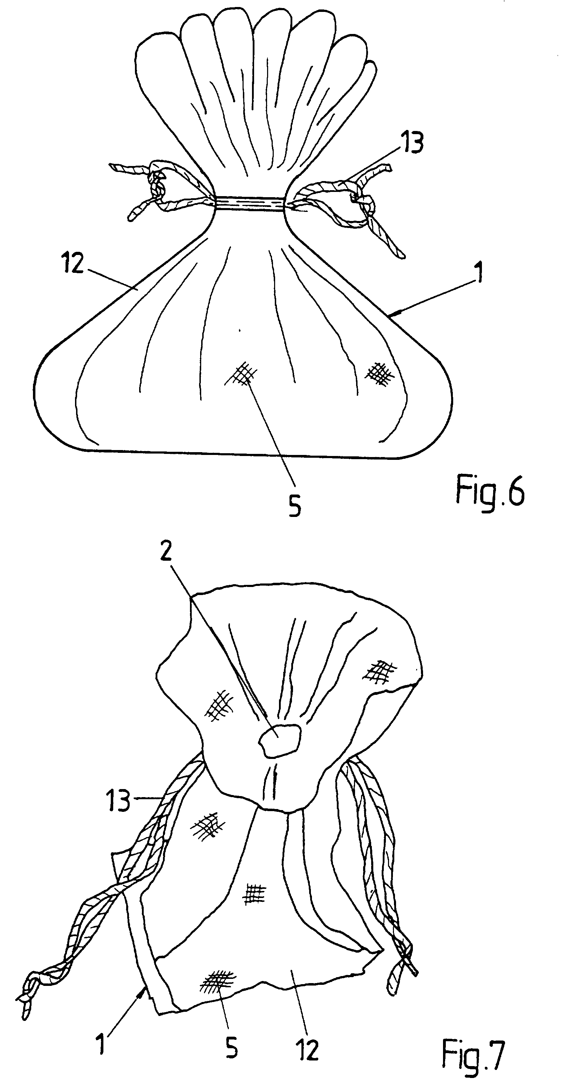

Die Figuren 6 und 7 zeigen ein weiteres Ausführungsbeispiel einer erfindungsgemäßen Vorrichtung, wobei die Hülle 1 als Beutel 12 ausgebildet ist und als Verschluß einen Kordelzug 13 aufweist.

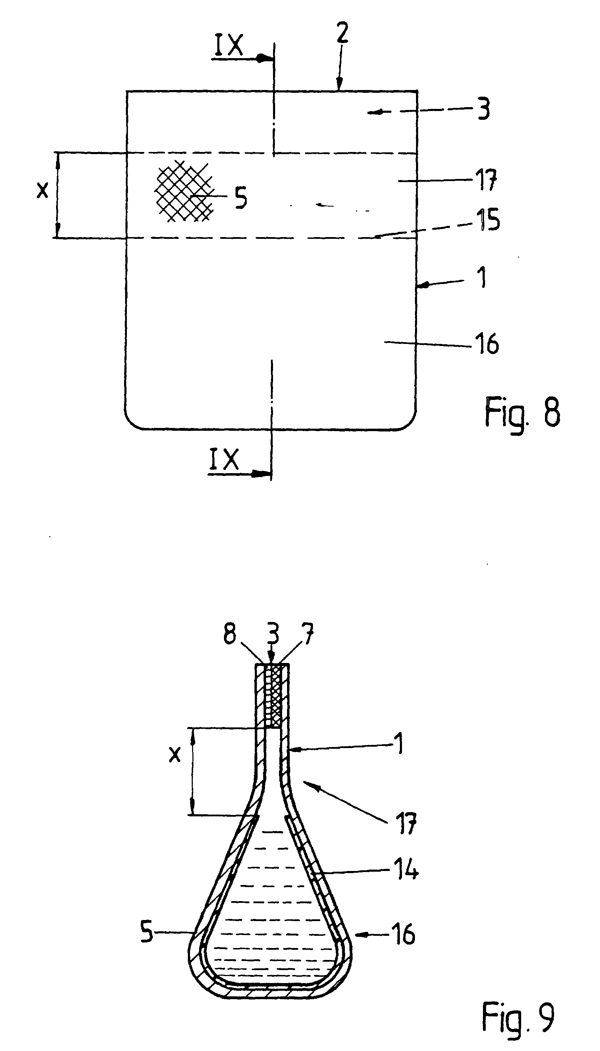

Während in den Ausführungsbeispielen der Figuren 1 - 7 der als Speicher für das flüssige Wäschebehandlungsmittel dienende Meßbecher 4 in das Innere der Hülle 1 eingebracht werden muß, ist nach den nun folgenden Ausführungsbeispielen der Figuren 8 und 9 dieser Einsetzvorgang nicht erforderlich. Der Speicher ist hier von einer behälterartig ausgebildeten, flüssigkeitsundurchlässigen Folie 14 gebildet, die an der Innenwand der beutelartigen Hülle 1 angeordnet ist. In dem dargestellten Ausführungsbeispiel ist die Hülle 1 "gemischtdurchlässig" gestaltet, das heißt, das Unterteil ist undurchlässig für die Wäschebehandlungsflüssigkeit, während das Oberteil durchlässig ist. Dieses folgt daraus, daß sich die Folie 14 nicht bis zu dem Klettverschluß 3 der Hülle 1 erstreckt, sondern hier den Abstand x beläßt.

In der Fig. 8 deutet die gestrichelt eingezeichnete Linie 15 den Übergang von mit Folie 14 versehenem und folienfreiem Bereich der Hülle 1 an. Der Bodenbereich 16 ist - wie aus Fig. 9 ersichtlich - mit der Folie 14 versehen, während der an den Verschluß 3 angrenzende Bereich 17 keine Folie 14 aufweist, sondern nur aus der offenporigen Zellstruktur 5 besteht.

Für den Einsatz wird der Klettverschluß 3 der erfindungsgemäßen Vorrichtung geöffnet und anschließend das flüssige Wäschebehandlungsmittel in das Innere des Beutels gegossen. Dabei verhindert die Folie 14, daß es aus dem Boden der Vorrichtung austritt. Bei Verwendung entsprechend dünner oder durchscheinender Folien kann durch entsprechend aufgebrachte Markierungen der Dosiervorgang korrekt durchgeführt werden. Gegebenenfalls kann die Hülle 1 hierzu mit einem Sichtfenster versehen sein. Ist die Dosierung abgeschlossen, so wird der Klettverschluß 3 wieder verschlossen und die erfindungsgemäße Vorrichtung zusammen mit der zu behandelnden Wäsche in die Trommel einer Waschmaschine oder dergleichen gepackt. Durch die Trommelbewegung gelangt das flüssige Wäschebehandlungsmittel in den Bereich x der Hülle 1 und kann dort durch die offenporige, saugfähige Zellstruktur 5 nach und nach austreten.

Nach einem nicht dargestellten Ausführungsbeispiel ist es auch möglich, den gesamten inneren Bereich der Hülle 1 mit Folie 14 zu versehen, wobei dann der Verschluß der Hülle 1 derart ausgebildet sein muß, daß er ein Austreten der Wäschebehandlungsfüssigkeit zuläßt. Dieses kann durch die Walkbewegung hervorgerufen werden, indem durch die hierdurch erfolgende Krafteinwirkung nach und nach der Hülleninhalt durch den nicht völlig dicht schließenden Verschluß herausgepreßt wird. Wird ein temperaturabhängiger Kleber zum Schließen des Verschlusses eingesetzt, so öffnet dieser, sobald das Waschwasser die entsprechende Temperatur erreicht hat. Die Folie 14 kann auch als Beschichtung der Hülle 1 ausgebildet sein. Bei Einsatz bestimmter Materialien kann sie auch von einer geschlossenen Haut dieser Materialien gebildet werden. Zum Einsatz gelangen kann auch ein flexibler, weicher, kochbeständiger Kunststoff z. B. Santoprene oder Vestoprene.

Selbstverständlich liegt es im Rahmen der Erfindung, die Form der Hülle unterschiedlich zu gestalten. Ferner ist es möglich, die Hülle und gegebenenfalls auch die Folie mit Mengenmarkierungen, Gebrauchsanweisungen sowie Werbehinweisen und dergleichen zu bedrucken. Ferner kann die Hülle so gestaltet sein, daß sich ein Zweitnutzen ergibt, der z. B. darin besteht, den Beutel als Waschhandschuh oder auch als Behälter für Seife zu benutzen.

Wird für die Hülle Schaumstoff verwendet, so bietet sich insbesondere solches Material an, welches bis mindestens 95° C temperaturbeständig ist. Vorzugsweise wird offenporiger Polyurethan-Schaum mit einer Dicke von 1 - 6mm verwendet. Einerseits kann in die Hülle aus offenporigem Schaumstoff ein entsprechender Speicher eingesetzt werden. Alternativ ist es auch möglich, daß der Speicher selbst durch ein verschließbares Behältnis aus offenporigem Schaumstoff gebildet ist. Ein solches Schaumstoff-Behältnis eignet sich zur Aufnahme von Waschpulver, ohne daß ein spezieller, das Waschmittel aufnehmender Behälter vorgesehen sein müßte. Bei einer Schaumstoff-Dicke von 2mm löst sich das Waschpulver nach Einlegen des Behältnisses zwischen die Wäsche und Zugabe von Waschwasser in 2 - 3min. auf, wenn von offenporigem Polyurethan-Schaumstoff mit entsprechender Porengröße ausgegangen wird.

Gemäß der Darstellung in den Fig. 10 und 11 ist die Hülle 1 von einer Schaumstofftasche 20 gebildet. Als Material ist Polyurethan-Schaumstoff gewählt mit einer Dicke von ca. 3mm. Die Schaumstofftasche 20 weist zwei rechteckförmige, deckend zueinander angeordnete, an drei Randseiten 21, 22, 23 miteinander verbundene Taschenwände 24 und 25 auf. Von der rückwärtigen Taschenwand 25 geht eine umgelegte, die Taschenöffnung 26 überfangende Verschlußlasche 27 aus. Die Schmalkanten 28 sind mit den entsprechenden Bereichen der Randseiten 21, 22 verbunden. Die Verbindung der Randseiten zusammen mit der jeweils zugehörigen Schmalkante 28 erfolgt durch Verschweißung. Hierzu bietet sich insbesondere eine Hochfrequenzverschweißung an, wobei die entsprechenden Randbereiche der Schaumstofftasche miteinander verschmelzen.

Insbesondere geht aus Fig. 11 hervor, daß die Verschlußlasche 27 einstückig mit der zugehörigen Taschenwand 25 ausgebildet ist. Anstatt wie dargestellt ist, könnte sich diese rückwärtige Taschenwand 25 einstückig in die vorderseitige Taschenwand 24 fortsetzen, so daß die Schaumstofftasche aus einem einzigen Zuschnitt gefertigt werden kann.

Beim Ausführungsbeispiel besitzt die Schaumstofftasche 20 ein rechteckiges Format von etwa 20cm Länge L, während die Breite B 15cm beträgt. Die Taschenöffnung 26 befindet sich an der der Randseite 23 gegenüberliegenden Breitseite der Schaumstofftasche. Als Überstand Ü der Verschlußlasche 27 über die Taschenöffnung 26 sind 4cm gewählt. Das Beschicken des Tascheninneren 29 erfolgt in der Weise, daß die vordere Taschenwand 24 über die Verschlußlasche 27 hinausgezogen wird unter Bildung einer Einfüllöffnung. Nach dem Einfüllvorgang ist dann die Verschlußlasche 27 in ihre überfangende Lage zu bringen, so daß eine Selbstentleerung des in die Schaumstofftasche eingebrachten Waschpulvers verhindert ist.

Die Schaumstofftasche 20 kann bei zurückgeklappter Verschlußlasche 27 als Waschhandschuh, z.B. für das Säubern von harten Oberflächen, benutzt werden. Die verschlossene Schaumstofftasche 20 läßt sich ferner im Nebennutzen als Wäschesäckchen für das Waschen empfindlicher Kleintextilen, z.B. Damenfeinstrümpfe oder Strumpfhosen, benutzen.

Alle in der Beschreibung erwähnten und in der Zeichnung dargestellten neuen Merkmale sind erfindungswesentlich, auch soweit sie in den Ansprüchen nicht ausdrücklich beansprucht sind.

## Patentansprüche

1. Dosiervorrichtung zur Aufnahme eines flüssigen oder pulverförmigen Wäschebehandlungsmittels und zur Abgabe einer sich aus dieser und zufließendem Waschwasser und/oder zufließender Waschflotte bildenden Wäschebehandlungsflüssigkeit innerhalb einer Waschmaschine, welche Dosiervorrichtung aus einer Hülle (1) mit Einfüllöffnung (2) besteht, **dadurch gekennzeichnet, dass** die Wandung der beutel- oder taschenförmig ausgebildeten Hülle aus flexibler und offenporiger Struktur aus Gewebe, Gewirke, Vlies oder Schaumstoff besteht und eine Rückhaltefähigkeit besitzt, derart, dass das Wäschebehandlungsmittel nicht direkt austreten kann, jedoch verdünnte Wäschebehandlungsflüssigkeit aus der Hülle herausgespült werden kann.

2. Dosiervorrichtung nach Anspruch 1, **dadurch gekennzeichnet, dass** die Hülle (1) als kuvertartige Tasche (9) ausgebildet ist.

3. Dosiervorrichtung nach Anspruch 1 oder 2, **dadurch gekennzeichnet, dass** der Verschluss der Hülle (1) als Klettband (3), Kordelzug (13), Reißverschluss, elastisches Band oder insbesondere temperaturabhängig reagierender Kleberverschluss ausgebildet ist.

4. Dosiervorrichtung nach einem der Ansprüche 1 bis 3, **dadurch gekennzeichnet, dass** der Schaumstoff bis mindestens 95° C temperaturbeständig ist.

5. Dosiervorrichtung nach Anspruch 4, **dadurch gekennzeichnet, dass** der Schaumstoff offenporiger Polyurethanschaum ist.

6. Dosiervorrichtung nach einem der vorhergehenden Ansprüche, **dadurch gekennzeichnet, dass** die Dicke des Schaumstoffes 1 bis 6 mm, vorzugsweise 3 bis 4 mm, beträgt.

7. Dosiervorrichtung nach Anspruch 6, **dadurch gekennzeichnet, dass** bei Ausbildung einer Schaumstofftasche (20) zwei rechteckförmige, deckend zueinander angeordnete, an drei Randseiten (21, 22, 23) miteinander verbundene Taschenwände (24, 25) vorgesehen sind.

8. Dosiervorrichtung nach Anspruch 7, **dadurch gekennzeichnet, dass** von der einen Taschenwand (25) eine umgelegte, die Taschenöffnung (26) überfangende Verschlusslasche(27) ausgeht, deren Schmalkanten (28) mit den entsprechenden Bereichen der Randseiten (21, 22) verbunden sind.

9. Dosiervorrichtung nach Anspruch 8, **dadurch gekennzeichnet, dass** die Randseiten (21, 22, 23) zusammen mit der zugehörigen Schmalkante (28) verschweißt sind.

10. Dosiervorrichtung nach Anspruch 9, **dadurch gekennzeichnet, dass** die Randseiten (21, 22, 23) zusammen mit der zugehörigen Schmalkante (28) miteinander hochfrequenzverschweißt sind.

11. Dosiervorrichtung nach einem der Ansprüche 8 bis 10, **dadurch gekennzeichnet, dass** die Verschlusslasche (27) einstückig mit der zugehörigen Taschenwand (25) ausgebildet ist.

12. Dosiervorrichtung nach einem der Ansprüche 7 bis 11, **dadurch gekennzeichnet, dass** die Schaumstofftasche (20) ein Format von etwa 20 cm Länge (L) und 15 cm Breite (B) aufweist.

13. Dosiervorrichtung nach einem der Ansprüche 7 bis 12, **dadurch gekennzeichnet, dass** die Taschenöffnung (26) an der einen Breitseite der Schaumstofftasche (20) ausgebildet ist.

14. Dosiervorrichtung nach einem der Ansprüche 8 bis 13, **dadurch gekennzeichnet, dass** die Verschlusslasche (27) einen Überstand (Ü) über die Taschenöffnung (26) von etwa 3 bis 6 cm aufweist.

## Claims

1. Metering device for accommodating a liquid or powder-foam washing agent and for delivering, within a washing machine, a washing agent liquid formed from the liquid or powder-foam washing agent and inflowing washing water and/or inflowing detergent solution, which metering device consists of an enclosure (1) with a filling opening (2), **characterised in that** the wall of the bag or pouch-foam enclosure consists of a flexible or open-pored structure of a woven, knitted, non-woven or foam material and has a retention capability such that the washing agent cannot exit directly, but diluted washing agent liquid can be flushed out of the enclosure.

2. Metering device according to Claim 1, **characterised in that** the enclosure (1) is formed as an envelope-type pouch.

3. Metering device according to Claim 1 or 2, **characterised in that** the closure of the enclosure (1) is formed as a Velcro strip (3), a pull cord (13), a tear closure, an elastic band, or in particular as a temperature responsive adhesive closure.

4. Metering device according to one of Claims 1 to 3, **characterised in that** the foam material is temperature resistant up to least 95°C.

5. Metering device according to Claim 4, **characterised in that** the foam material is an open-pored polyurethane foam.

6. Metering device according to one of the preceding claims, **characterised in that** the thickness of the foam material is 1 to 6 mm, preferably 3 to 4 mm.

7. Metering device according to Claim 6, **characterised in that** forming a foam material pouch (20), two rectangular pouch walls (24, 25) are provided, which are arranged to be coincident with one another and are connected to each other on three edges (21, 22, 23).

8. Metering device according to Claim 7, **characterised in that** from one (25) of the walls of the pouch, there extends a closure tongue (27) which is turned down and engages over the opening (26) of the pouch, the narrow edges (28) of the tongue being connected to the corresponding regions of the side edges (21, 22).

9. Metering device according to Claim 8, **characterised in that** the edges (21, 22, 23), together with the associated narrow edge (28), are welded.

10. Metering device according to Claim 9, **characterised in that** the edges (21, 22, 23) together with the associated narrow edge (28), are high-frequency-welded to one another.

11. Metering device according to one of Claims 8 to 10, **characterised in that** the closure tongue (27) is formed in one piece with the associated pouch wall (25).

12. Metering device according to one of Claims 7 to 11, **characterised in that** the foam material pouch (20) is, in size, about 20 cm long (L) and 15 cm wide (B).

13. Metering device according to one of Claims 7 to 12, **characterised in that** the pouch opening (26) is formed on one of the wide sides of the foam material pouch (20).

14. Metering device according to one of Claims 8 to 13, **characterised in that** the closure tongue (27) has a projection (Ü) of approximately 3 to 6 cm beyond the pouch opening (26).

## Revendications

1. Dispositif de dosage pour recevoir un moyen liquide ou pulvérulent de traitement par lavage et pour délivrer un liquide de traitement par lavage formé à partir de ce moyen et d'eau de lavage en écoulement et/ou d'une quantité de liquide de lavage en écoulement à l'intérieur d'une machine à laver, lequel dispositif de dosage se compose d'une enveloppe (1) avec une ouverture de remplissage (2), **caractérisé en ce que** la paroi de l'enveloppe réalisée en forme de sachet ou de poche se compose d'une structure flexible et à pores ouverts en tissu, en tricoté, en non tissé ou en matière alvéolaire, et présente une capacité de retenue, de sorte que le moyen de traitement par lavage ne puisse pas sortir directement, mais que l'on puisse cependant refouler hors de l'enveloppe le liquide de traitement par lavage.

2. Dispositif de dosage selon la revendication 1, **caractérisé en ce que** l'enveloppe (1) est réalisée comme une poche (9) du genre d'une enveloppe pour le courrier.

3. Dispositif de dosage selon la revendication 1 ou 2, **caractérisé en ce que** la fermeture de l'enveloppe (1) est réalisée sous la forme d'une bande à auto-accrochage (3), d'un cordon de tirage (13) de fermeture à glissière, d'une bande élastique ou en particulier d'une fermeture par collage réagissant en fonction de la température.

4. Dispositif de dosage selon l'une des revendications 1 à 3, **caractérisé en ce que** la matière alvéolaire est résistante à la température jusqu'à 95 °C.

5. Dispositif de dosage selon la revendication 4, **caractérisé en ce que** la matière alvéolaire est de la mousse de polyuréthanne à pores ouverts.

6. Dispositif de dosage selon l'une des revendications précédentes, **caractérisé en ce que** l'épaisseur de la matière alvéolaire atteint 1 à 6 mm, de préférence de 3 à 4 mm.

7. Dispositif de dosage selon la revendication 6, **caractérisé en ce qu'**à la formation d'une poche (20) en matière alvéolaire, sont prévues deux parois de poche (24, 25) de forme rectangulaire disposées recouvrantes l'une par rapport à l'autre et reliées l'une à l'autre sur trois côtés de bordure (21, 22, 23).

8. Dispositif de dosage selon la revendication 7, **caractérisé en ce que** une languette de fermeture (17) repliée et recouvrant l'ouverture de poche (26) s'étend à partir de l'une (25) des parois de poche, et dont les bords étroits (28) sont reliés aux zones correspondantes des côtés de bordure (21, 22).

9. Dispositif de dosage selon la revendication 8, **caractérisé en ce que** les côtés de bordure (21, 22, 23) sont soudés ensemble avec le bord étroit correspondant (28).

10. Dispositif de dosage selon la revendication 9, **caractérisé en ce que** les côtés de bordure (21, 22, 23) sont soudés ensemble à haute fréquence avec le bord étroit correspondant (28).

11. Dispositif de dosage selon l'une des revendications 8 à 10, **caractérisé en ce que** la languette de fermeture (27) est réalisée monobloc avec la paroi de poche (25) correspondante.

12. Dispositif de dosage selon l'une des revendications 7 à 11, **caractérisé en ce que** la poche (20) en matière alvéolaire présente un format d'environ 20 cm de longueur (L) et 15 cm de largeur (B).

13. Dispositif de dosage selon l'une des revendications 7 à 12, **caractérisé en ce que** l'ouverture de poche (26) est réalisée sur l'une des faces larges de la poche (20) en matière alvéolaire.

14. Dispositif de dosage selon l'une des revendications 8 à 13, **caractérisé en ce que** la languette de fermeture (27) présente un dépassement (Ü) sur l'ouverture de poche (26) d'environ 3 à 6 cm.
